# EUROPEAN PATENT SPECIFICATION

(11) **EP 3 720 581 B1**
(45) Date of publication and mention of the grant of the patent: **20.11.2024**
(21) Application number: 18886327.8
(22) Date of filing: 05.12.2018
(51) Int. Cl.: G09B 5/06, G09B 17/00, G09B 19/04, G09B 19/06, A63F 13/60, A63F 13/80, A61B 5/16, A63F 13/79, G16H 20/70

(54) **SYSTEM AND METHOD FOR DEVICE-BASED COGNITIVE DEVELOPMENT OR THERAPY**
SYSTEM UND VERFAHREN ZUR VORRICHTUNGSBASIERTEN KOGNITIVEN ENTWICKLUNG ODER THERAPIE
SYSTÈME ET PROCÉDÉ DE DÉVELOPPEMENT COGNITIF, OU DE THÉRAPIE COGNITIIVE, BASÉ SUR UN DISPOSITIF

(30) Priority: 05.12.2017 US 201762594595 P
(43) Date of publication of application: 14.10.2020
(73) Proprietor: Orange Neurosciences Corporation, Kingston, Ontario K7P 2A4 (CA)
(72) Inventor: SINGH, Vinay Kumar, Kingston Ontario K7P 2A4 (CA)
(74) Representative: Herrero & Asociados, S.L.
(86) International application number: PCT/CA2018/051560
(87) International publication number: WO 2019/109187

(56) References cited:
- WO-A1-2014/078902
- US-A1- 2009 069 707
- US-A1- 2009 069 707
- US-A1- 2016 240 098

## Description

### CROSS-REFERENCE

### FIELD

The present disclosure relates to systems and methods for providing device-based cognitive development or therapy, including but not limited to systems and methods for providing device-based personalized games and exercises assisting in development or providing a therapeutic benefit.

### BACKGROUND

Reading, writing and speech are critical abilities for success in life. However, many people have some form of learning difference, or learning disability, which hinders learning and development. Additionally, even in the absence of a learning disability, many people, including children, benefit from practice or exercises to improve or accelerate learning or comprehension with respect to reading, writing or speech, whether in their native language or a new language being learned.

Various tools such as verbal word games, flash cards, and other paper and pencil games exist to assist a person in learning, and to assist in the development of working memory, which plays an important role in reading, writing and speech. Some computerized games and approaches also exist.

Document US2009069707 discloses a neurofeedback training system that utilizes EEG activity based goals to help motivate trainees to learn to produce and sustain desirable EEG activity by enabling them to earn "game play" time.

Improvements in systems and methods for device-based cognitive therapy or device-based learning or development are desirable.

### BRIEF DESCRIPTION OF THE DRAWINGS

Embodiments of the present disclosure will now be described, by way of example only, with reference to the attached Figures.
Figure 1 is a block diagram of a system for providing a device-based multi-sensory therapeutic game according to an embodiment of the present disclosure.
Figure 2 is a block diagram of a system for providing a device-based multi-sensory therapeutic game according to an example embodiment of the present disclosure.
Figure 3 is a flowchart illustrating a method for providing a device-based multi-sensory therapeutic game according to an embodiment of the present disclosure.
Figure 4 is a block diagram illustrating orthographic and phonological components that are targeted to improve working memory and/or executive function according to an example embodiment of the present disclosure.
Figures 5, 6 and 7 are graphs illustrating testing improvement results from users employing a system or method according to an example embodiment of the present disclosure.
Figures 8, 9, 10 and 11 are visual representations and screenshots illustrating example visual outputs, or display examples, of device-based multi-sensory therapeutic games according to embodiments of the present disclosure.

### BRIEF DESCRIPTION

The present invention relates to a system for providing a device based multi sensory therapeutic game as defined in independent claim 1 and to a method for providing a device based multi sensory therapeutic game as defined in independent method claim 7.

In an embodiment, the present disclosure provides a system for providing a device-based multi-sensory therapeutic game comprising: a component provision module, in communication with a database, configured to provide game components and a game generation framework; a generator module, in communication with the component provision module, configured to generate, in real-time and using the game generation framework in conjunction with the database, scenes and assets including auditory game objects and visual game objects based on personalized user parameters; and a display module configured to display, in real-time to the user, a game that concurrently challenges the user's visual pathway, auditory pathway and cognitive pathway to create neuroplasticity, the game including the game components, the auditory game objects and the visual game objects.

In an example embodiment, the game components displayed by the display module comprise generic game components that are presented in a similar manner to any user, and the visual game objects and the auditory game objects comprise components that are based on personalized user parameters, and are presented in a different manner to different users depending on the associated personalized user parameters.

In an example embodiment, the component provision module, the generator module and the display module cooperate to provide the device-based multi-sensory therapeutic game adapted to provide therapy for attention deficit hyperactivity disorder (ADHD).

In an example embodiment, the scenes and assets including the auditory game objects and the visual game objects are generated independent of a language of game delivery.

In an example embodiment, the game components, the auditory game objects and the visual game objects are provided in a first language to concurrently challenge the user's visual pathway, auditory pathway and cognitive pathway in the first language.

In an example embodiment, the game components, the auditory game objects and the visual game objects are provided in a first language to concurrently challenge the user's visual pathway, auditory pathway and cognitive pathway in a second language, the second language being different from the first language.

In another embodiment, the present disclosure provides a processor-implemented method for providing a device-based multi-sensory therapeutic game comprising: obtaining, from a user condition database, personalized user parameters associated with a condition of the user for which the multi-sensory therapeutic game is designed to provide therapy; generating, in real-time and using a game generation framework in conjunction with a game database, scenes and assets including auditory game objects and visual game objects based on the personalized user parameters; and displaying, in real-time to the user, a game that concurrently provides a visual pathway challenge, an auditory pathway challenge and a cognitive pathway challenge to encourage neuroplasticity, the game including the auditory game objects and the visual game objects.

In an example embodiment, the displayed game components comprise generic game components that are presented in a similar manner to any user, and the displayed visual game objects and the displayed auditory game objects comprise components that are based on personalized user parameters, and are presented in a different manner to different users depending on the associated personalized user parameters.

In an example embodiment, displaying the game comprises simultaneously providing the visual pathway challenge, the auditory pathway challenge, and the cognitive pathway challenge.

In an example embodiment, simultaneously providing the visual pathway challenge, the auditory pathway challenge, and the cognitive pathway challenge comprises using complete words and their corresponding sounds.

In an example embodiment, displaying the game comprises displaying both translucent and opaque moving masks to induce attention.

In an example embodiment, displaying the game comprises providing a cognition inducing component selected from the group consisting of rhymes, pidgin English and homophones.

In an example embodiment, displaying the game comprises interspersing repetitive language tasks with non-language mosaics.

In an example embodiment, displaying the game comprises displaying motion graphics with embedded text.

In an example embodiment, displaying the game comprises providing a reward comprising visual and aural feedback.

In an example embodiment, displaying the game further comprises modifying at least one aspect of the displayed game based on a modification in one or more of the personalized user parameters.

In an example embodiment, displaying the game further comprises modifying at least one aspect of the displayed game based on a measured result of the user in an evaluated portion of the game.

In an example embodiment, modifying the at least one aspect of the displayed game is performed based on a relationship of the measured result to either: an expected result for the user based on one or more of the personalized user parameters; or a game modification threshold.

In an example embodiment, displaying the game comprises displaying fast motion graphics in addition to the auditory game objects and the visual game objects.

In an example embodiment, the method further comprises: creating the user condition database based on providing a pre-assessment exercise to the user and generating user condition data based on the user's results of performing the pre-assessment exercise.

In an example embodiment, displaying the game comprises providing the auditory pathway challenge to address one or more phonological components.

In an example embodiment, displaying the game comprises providing the auditory pathway challenge to address pronunciation.

In an example embodiment, displaying the game comprises providing the visual pathway challenge to address one or more orthographic aspects.

In an example embodiment, displaying the game comprises providing the visual pathway challenge to address spelling.

In an example embodiment, displaying the game comprises providing the cognitive pathway challenge to address one or more motor or working memory aspects.

In an example embodiment, displaying the game comprises providing the cognitive pathway challenge to address holding and processing information for a brief period of time.

In an example embodiment, the obtaining, the generating and the displaying cooperate to provide the device-based multi-sensory therapeutic game adapted to provide therapy for attention deficit hyperactivity disorder (ADHD).

In an example embodiment, the scenes and assets including the auditory game objects and the visual game objects are generated independent of a language of game delivery.

In an example embodiment, the game components, the auditory game objects and the visual game objects are displayed in a first language to concurrently challenge the user's visual pathway, auditory pathway and cognitive pathway in the first language.

In an example embodiment, the game components, the auditory game objects and the visual game objects are displayed in a first language to concurrently challenge the user's visual pathway, auditory pathway and cognitive pathway in a second language, the second language being different from the first language.

In an embodiment, the present disclosure provides a machine-readable memory storing statements and instructions for execution by the one or more processors to perform the method according to one or more embodiments as described herein.

In another embodiment, the present disclosure provides an apparatus for providing a device-based multi-sensory therapeutic game comprising: one or more processors; and a machine-readable memory storing statements and instructions for execution by the one or more processors to perform the method according to one or more embodiments as described herein.

In a further embodiment, the present disclosure provides a system for providing a device-based multi-sensory learning development tool comprising: a component provision module, in communication with a database, configured to provide game components and a game generation framework; a generator module, in communication with the component provision module, configured to generate, in real-time and using the game generation framework in conjunction with the database, scenes and assets including auditory game objects and visual game objects based on personalized user parameters; and a display module configured to display, in real-time to the user, a learning development game that concurrently challenges the user's visual pathway, auditory pathway and cognitive pathway to create neuroplasticity, the game including the game components, the auditory game objects and the visual game objects.

In an example embodiment, the game components displayed by the display module comprise generic game components that are presented in a similar manner to any user, and the visual game objects and the auditory game objects comprise components that are based on personalized user parameters, and are presented in a different manner to different users depending on the associated personalized user parameters.

In an example embodiment, the component provision module, the generator module and the display module cooperate to provide the device-based multi-sensory learning development tool adapted to provide a learning development game adapted for attention deficit hyperactivity disorder (ADHD).

In an example embodiment, the scenes and assets including the auditory game objects and the visual game objects are generated independent of a language of learning development tool delivery.

In an example embodiment, the game components, the auditory game objects and the visual game objects are provided in a first language to concurrently challenge the user's visual pathway, auditory pathway and cognitive pathway in the first language.

In an example embodiment, the game components, the auditory game objects and the visual game objects are provided in a first language to concurrently challenge the user's visual pathway, auditory pathway and cognitive pathway in a second language, the second language being different from the first language.

In another embodiment, the present disclosure provides a processor-implemented method for providing a device-based multi-sensory learning development tool comprising: obtaining, from a user condition database, personalized user parameters associated with a condition of the user for which the multi-sensory therapeutic game is designed to provide therapy; generating, in real-time and using a game generation framework in conjunction with a game database, scenes and assets including auditory game objects and visual game objects based on the personalized user parameters; and displaying, in real-time to the user, a learning development game that concurrently provide a visual pathway challenge, an auditory pathway challenge and a cognitive pathway challenge to encourage neuroplasticity, the game including the auditory game objects and the visual game objects.

In an example embodiment, the displayed game components comprise generic game components that are presented in a similar manner to any user, and the displayed visual game objects and the displayed auditory game objects comprise components that are based on personalized user parameters, and are presented in a different manner to different users depending on the associated personalized user parameters.

In an example embodiment, the obtaining, the generating and the displaying cooperate to provide the device-based multi-sensory learning development tool adapted to provide a learning development game adapted for attention deficit hyperactivity disorder (ADHD).

In an example embodiment, the scenes and assets including the auditory game objects and the visual game objects are generated independent of a language of game delivery.

In an example embodiment, the game components, the auditory game objects and the visual game objects are displayed in a first language to concurrently challenge the user's visual pathway, auditory pathway and cognitive pathway in the first language.

In an example embodiment, the game components, the auditory game objects and the visual game objects are displayed in a first language to concurrently challenge the user's visual pathway, auditory pathway and cognitive pathway in a second language, the second language being different from the first language.

In another embodiment, the present disclosure provides a machine-readable memory storing statements and instructions for execution by the one or more processors to perform the method according to one or more embodiments as described herein.

In a further embodiment, the present disclosure provides an apparatus for providing a device-based multi-sensory learning development tool comprising: one or more processors; and a machine-readable memory storing statements and instructions for execution by the one or more processors to perform the method of one or more embodiment as described herein.

In additional embodiments, the present disclosure provides a system, apparatus, processor-implemented method, or machine-readable memory comprising a combination of features comprising a subset or a different combination of features than the features or set of features explicitly recited herein.

In an embodiment, the present disclosure provides a system for providing a device-based multi-sensory therapeutic game comprising: a component provision module, in communication with a database, configured to provide a game generation framework; a generator module, in communication with the component provision module, configured to generate, in real-time and using the game generation framework in conjunction with the database, scenes and assets including auditory game objects and visual game objects based on personalized user parameters; and a display module configured to display, in real-time to the user, a game that concurrently provides a visual pathway challenge, an auditory pathway challenge and a cognitive pathway challenge to encourage neuroplasticity, the game including the auditory game objects and the visual game objects.

In another embodiment, the present disclosure provides a processor-implemented method for providing a device-based multi-sensory therapeutic game comprising: obtaining, from a user condition database, personalized user parameters associated with a condition of the user for which the multi-sensory therapeutic game is designed to provide therapy; generating, in real-time and using a game generation framework in conjunction with a game database, scenes and assets including auditory game objects and visual game objects based on the personalized user parameters; and displaying, in real-time to the user, a game that concurrently provides a visual pathway challenge, an auditory pathway challenge and a cognitive pathway challenge to encourage neuroplasticity, the game including the auditory game objects and the visual game objects.

In a further embodiment, the present disclosure provides a system for providing a device-based multi-sensory learning development tool comprising: a component provision module, in communication with a database, configured to provide a game generation framework; a generator module, in communication with the component provision module, configured to generate, in real-time and using the game generation framework in conjunction with the database, scenes and assets including auditory game objects and visual game objects based on personalized user parameters; and a display module configured to display, in real-time to the user, a learning development game that concurrently provides a visual pathway challenge, an auditory pathway challenge and a cognitive pathway challenge to encourage neuroplasticity, the game including the auditory game objects and the visual game objects.

In another embodiment, the present disclosure provides a processor-implemented method for providing a device-based multi-sensory learning development tool comprising: obtaining, from a user condition database, personalized user parameters associated with a condition of the user for which the multi-sensory therapeutic game is designed to provide therapy; generating, in real-time and using a game generation framework in conjunction with a game database, scenes and assets including auditory game objects and visual game objects based on the personalized user parameters; and displaying, in real-time to the user, a learning development game that concurrently provide a visual pathway challenge, an auditory pathway challenge and a cognitive pathway challenge to encourage neuroplasticity, the game including the auditory game objects and the visual game objects.

### DETAILED DESCRIPTION

A method and system provide a device-based multi-sensory therapeutic game, or learning development tool. A component provision module, in communication with a database, is configured to provide game components and a game generation framework. A generator module is configured to generate, in real-time and using the game generation framework and the database, scenes and assets including auditory game objects and visual game objects based on personalized user parameters. A display module is configured to display, in real-time to the user, a game that concurrently challenges the user's visual pathway, auditory pathway and cognitive pathway to create neuroplasticity, the game including the game components, and the auditory and visual game objects. The methods and systems can provide one or more of the following advantages: improve auditory processing speed; improve visual processing speed; bond auditory and visual functions; increase working memory capacity; improve attention; improve motivation; and improve eye movement control.

For simplicity and clarity of illustration, reference numerals may be repeated among the figures to indicate corresponding or analogous elements. Numerous details are set forth to provide an understanding of the embodiments described herein. The embodiments may be practiced without these details. In other instances, well-known methods, procedures, and components have not been described in detail to avoid obscuring the embodiments described.

Embodiments of the present disclosure provide a system and method, such as an artificial intelligence based cognitive learning program, for foundations of language learning, such as applications for people with learning disabilities. Embodiments of the present disclosure create more efficient neural networks in brains of poor readers by targeting the auditory, visual and auditory-to-visual bridge through a combination of motion graphics, psychological games and acoustically modified sound.

Embodiments of the present disclosure bond the visual, auditory & motor functions relevant to reading. An accelerated neuroplasticity-based approach according to an embodiment of the present disclosure provides significant improvement in one or more of: word attack, word identification, reading rate, reading accuracy, reading comprehension and passage comprehension.

According to an embodiment of the present disclosure, a system is provided for the enhancement of the neurophysiological processes of a person, in particular those processes associated with reading, writing and speech. Embodiments of the present disclosure are applicable in the context of treating reading, writing and speech disorders. Other example embodiments have far broader applications including the treatment Aphasia or slowing dementia in Alzheimer's. A digital health solution according to an embodiment of the present disclosure provides a non-pharmacological treatment for a disease such as Aphasia or Alzheimer's. A computer based cognitive therapy program according to an embodiment of the present disclosure uses 2D/3D graphics, and optionally an augmented reality/virtual reality (AR/VR) environment to provide cognitive training to improve working memory and executive function.

Working memory refers to the ability to hold and process information in a person's mind for a brief period of time. Children use their working memory, for example when they are learning to match, or how to spell a word for the first time. Adults use working memory, for example when learning a new phone number or how to drive. Think of working memory as the brain's sticky note on which a person places information they need to hold onto and put into action.

Research shows a correlation between high working memory and success in school for a child. Issues with working memory can be correlated if a person is having hard time following instructions. The person seems to be daydreaming and not paying attention. This can also be result of an individual's working memory running out of space (think of a sticky note with no place to write) which can be due lack of attention and cognition (think of not able to write properly on the sticky note).

Research has shown that verbal word games, paper pencil games and some computerized games show improvement in working memories.

Executive functions refer to basic cognitive processes such as attentional control, cognitive inhibition, inhibitory control, working memory, and cognitive flexibility. Cognitive control is impaired in addiction, attention deficit hyperactivity disorder, and a number of other central nervous system disorders.

Embodiments of the present disclosure provide a system and method for delivering a cognitive therapy program that uses fast moving graphics, differential sounds and a gamified learning environment presented as an individualized program based on reading attributes of specific user. The program impacts/enhances the working memory and executive function through increase in attention and cognition of an individual.

Embodiments of the present disclosure provide a system and method that decrease visual processing and auditory processing deficits. Currently, most brain functions are tested and performed independently in therapy programs, taking a longer period of time and hence increasing stress, anxiety and depression in people, which negatively impacts working memory and hence becomes barrier to learning.

Embodiments of the present disclosure use accelerated neuroplasticity based approach where multiple pathways in a user's brain are targeted concurrently, or simultaneously.

Embodiments of the present disclosure provide a system and method for language learning in which a device-based multi-sensory therapeutic game is provided in any number of languages, with the underlying method, modules and components being the same for any language of program delivery, for example such that the therapy is language independent. For example, in an embodiment the same system and method can provide a device-based multi-sensory therapeutic game in English, Arabic, Hebrew, Spanish, French, Hindi or any other language, or variants/dialects of base languages.

A system and method according to an embodiment of the present disclosure provide a device-based multi-sensory therapeutic game for learning in a user's first language, or native language. In another embodiment, the system and method provide a device-based multi-sensory therapeutic game for learning a second or additional language. When providing for learning a second or additional language, the device-based multi-sensory therapeutic game can be provided in that same language (e.g. a French language game for learning French as a second language), or in another language (e.g. an English language game for learning French as a second language). Some traditional teachings have understood that a person wanting to learn in English should have instructions in English. However, there is some data showing that learning fluency and comprehension in French (or any second language) can be improved even though the instruction is in English (or any first language).

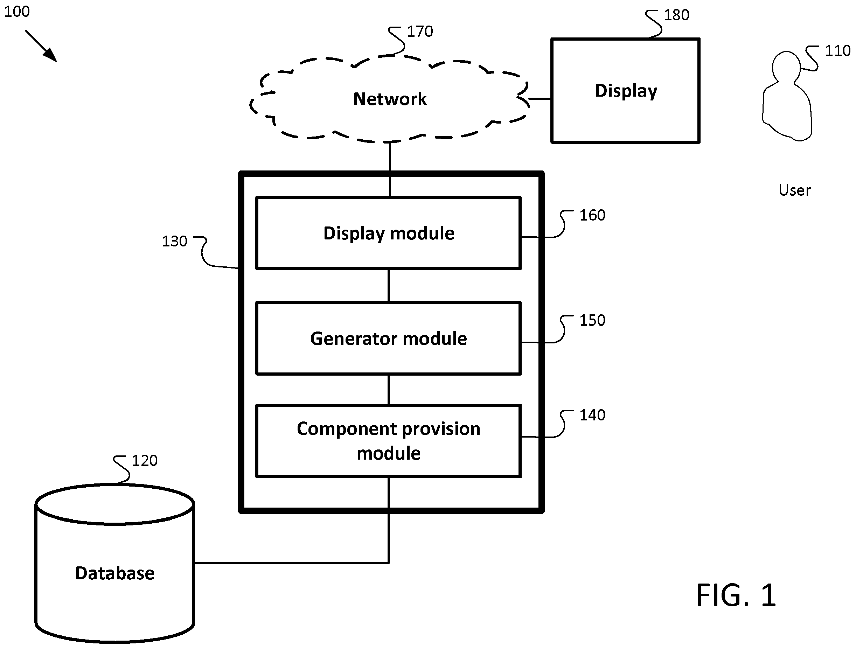

Figure 1 is a block diagram of a system for providing a device-based multi-sensory therapeutic game according to an embodiment of the present disclosure. In an environment 100, a user 110 can be described as having a learning profile, or learning parameters associated with the user. A database 120 stores personalized user parameters relating to the learning profile or learning parameters.

A system 130 for providing a device-based multi-sensory therapeutic game comprises: a component provision module 140; a generator module 150; and a display module 160. The component provision module, or backend engine, is in communication with the database 120, and configured to provide game components and a game generation framework. In an example embodiment, the game generation framework comprises a computer-readable memory storing statements and instructions for execution by a processor to produce elements or components of the device-based multi-sensory therapeutic game, for example based on stored templates. In an example embodiment, the game generation framework comprises stored logic and template components for generating the game. In an example embodiment, the game generation framework uses input parameters to generate game components and other aspects of the game based on the input parameters, for example by modifying a stored template component according to the input parameters. The generator module 150, or generator engine, is in communication with the component provision module 140, and configured to generate, in real-time and using the game generation framework in conjunction with the database, scenes and assets including auditory game objects and visual game objects based on personalized user parameters. The display module, or display engine, 160 is configured to display, in real-time to the user, a game that concurrently challenge the user's visual pathway, auditory pathway and cognitive pathway to create neuroplasticity, the game including the game components, the auditory game objects and the visual game objects. In an example embodiment, the game components comprise generic game components that are presented in a similar manner to any user; in an embodiment, the visual game objects and the auditory game objects comprise components that are based on personalized user parameters, and are presented in a different manner to different users depending on their personalized user parameters. In an example embodiment, the display module 160 is in communication with a display 180, optionally via a network 170 such as the Internet, to display the game and game components to the user 110.

In an example embodiment, the component provision module 140, or backend engine, comprises one or more of: a scripting language; an asset engine; a workflow engine; an artificial intelligence (bot) engine; a machine learning module; an application programming interface (API); a master data management module; and a data store (e.g. Hadoop, No-SQL, Relational).

In an example embodiment, an AI module in the component provision module 140 predicts/diagnoses impairments (or improvements) in the user and dynamically creates a therapy session in real-time by pulling different games from the database. This is similar to personalized medicine - or personalized non-pharmacological treatment for stopping or slowing memory loss in an aging individual or caused by dementia due to Alzheimer's. An increase in working memory and better eye-movement muscle control in stroke patients provides them the ability to read, as it is difficult for them to do manual reading exercises.

Further describing an example embodiment, a cognitive therapy platform according to an embodiment of the present disclosure generates personalized cognitive therapy in real-time. In an example implementation, the component provision module 140 comprises a custom API and uses data stored in the cloud in unstructured database, though any type of databases can be used. The component provision module 140 comprises an asset engine to generate audio sounds, videos, images, colors, 2D/3D objects which can be put to work in a workflow engine. In an embodiment, this is all configured using a machine learning module written specifically for this purpose. The machine learning module captures user's requirements through the interface, for example using a camera, microphone, touch or any sensory device tethered or untethered to the computer. In an embodiment, the system comprises the sensory device associated with the computer. In an example embodiment, the component provision module comprises the machine learning module.

In an example embodiment, an AI based BOT generator in the component provision module 140 uses all this information to write scripts which are transferred to various generators, such as provided in the generator module 150. In an example embodiment, the generators comprise a scene generator configured to develop scenes, and configured using a configurator, by utilizing various assets created by the asset generator. In an embodiment, a BOT generator feeds the script and a game generator is configured to output components of a therapy/game. In an example embodiment, a game comprises a program, different sessions at various levels and exercises built into the game to target auditory, visual and cognitive pathways in required fashion as identified though the UI. This real-time generated therapy is a unique way to develop personalized software therapy by utilizing machine learning and collecting data from the users in real-time.

In an example embodiment, the generator module 150 comprises one or more of: a scene generator; a configurator; an asset generator; a trigger generator; a bot generator; and a game generator. In an example embodiment, a scene in a displayed game comprises a plurality of specific asset definitions that trigger, or call, a plurality of generic asset definitions, such as objects, background, color, audio, video.

In an example embodiment, the display module 160 displays a game or exercise that comprises a program, sessions and exercises. In an example embodiment, the display module 160 interfaces with a user interface, which includes or provides one or more of: game modules; security; menus; and a management portal. In an example embodiment, the display module 160 interfaces with a deployment engine for deploying the display on any number of channels, such as Slack^{™}, Facebook^{™}, web, mobile, SMS, iOS^{™}, Android^{™}, Windows^{™}, Chrome OS^{™}, Linux, augmented reality (AR), mixed reality (MR) and virtual reality (VR) platforms.

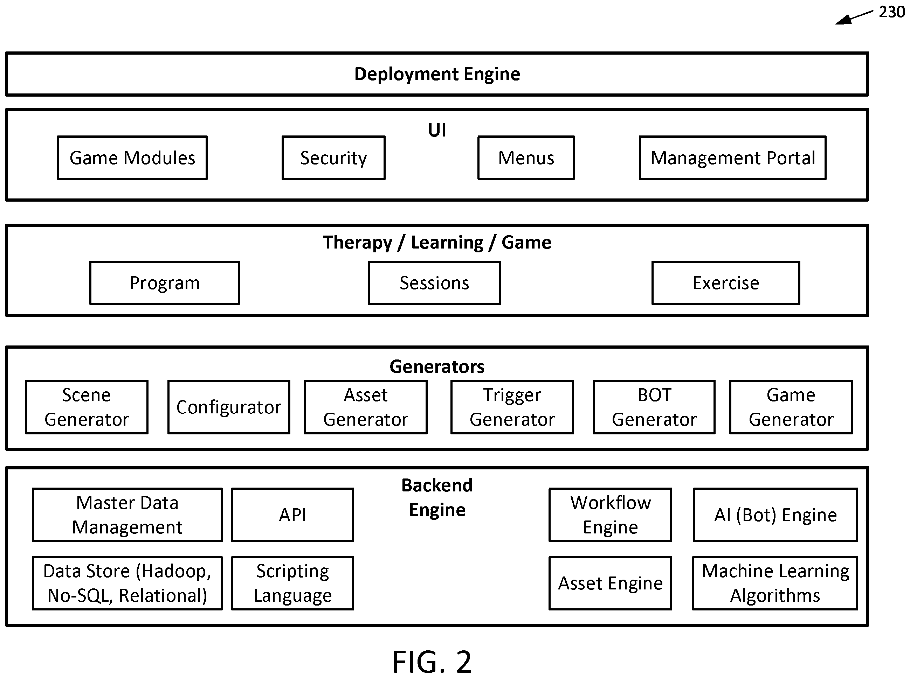

Figure 2 is a block diagram of a system for providing a device-based multi-sensory therapeutic game according to an example embodiment of the present disclosure. The system 230 in Figure 2 illustrates an example implementation of the system 130 of Figure 1, in which various examples discussed above are illustrated as examples. It is to be understood that embodiments of the present disclosure include variations of the system shown in Figure 2 that include fewer or more elements than those illustrated. For example, an embodiment of the present disclosure comprises only a subset of the elements illustrated in Figure 2. In an example embodiment, the generators of Figure 2 comprise an implementation of the generator module of Figure 1, and the backend engine of Figure 2 comprises an implementation of the component provision module of Figure 1.

Most known approaches provide a single app or reading card based manual tutoring program developed to help with reading only. Embodiments of the present disclosure use a game database with a large number of games with specific attributes configured to target visual processing, or auditory processing, or eye-hand coordination, or eye-ear or eye-hand-ear coordination or eye-hand-ear-legs (in VR and AR version). All these sessions are targeting to enhance working memory and executive function.

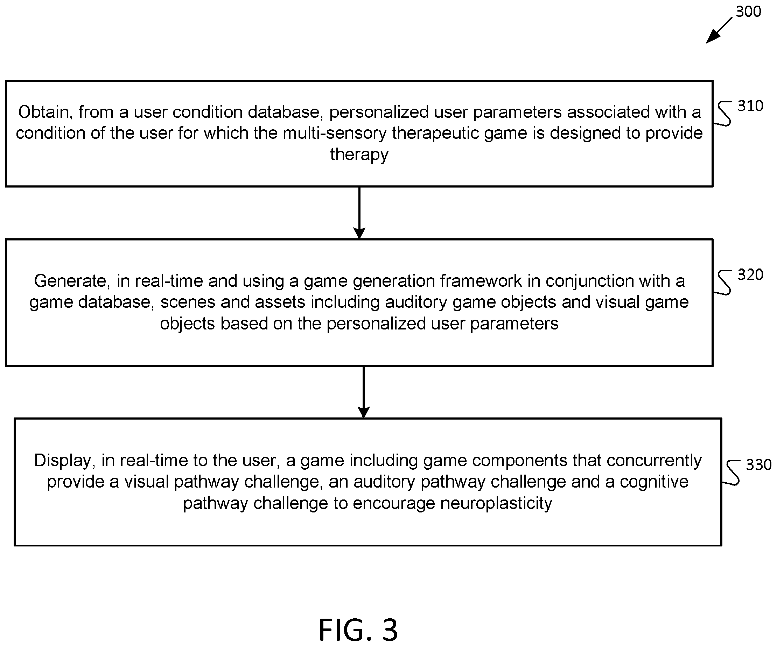

Figure 3 is a flowchart illustrating a processor-implemented method 200 for providing a device-based multi-sensory therapeutic game according to an embodiment of the present disclosure. The method 300 comprises: at 310, obtaining, from a user condition database, personalized user parameters associated with a condition of the user for which the multi-sensory therapeutic game is designed to provide therapy; at 320, generating, in real-time and using a game generation framework in conjunction with a game database, scenes and assets including auditory game objects and visual game objects based on the personalized user parameters; and, at 330, displaying, in real-time to the user, a game including game components that concurrently provide a visual pathway challenge, an auditory pathway challenge and a cognitive pathway challenge to encourage neuroplasticity.

In an example embodiment: step 310 in Figure 3 is performed by the component provision module in Figure 1; step 320 in Figure 3 is performed by the generator module 150 in Figure 1; and step 330 in Figure 3 is performed by the display module 160 in Figure 1.

In an example embodiment, displaying the game comprises simultaneously providing the visual pathway challenge, the auditory pathway challenge, and the cognitive pathway challenge. In an example embodiment, simultaneously providing the visual pathway challenge, the auditory pathway challenge, and the cognitive pathway challenge comprises using complete words and their corresponding sounds. For example, in an embodiment, the system or method according to an embodiment of the present disclosure provides an exercise or game that simultaneously targets auditory, visual and motor skills.

In an implementation, a system or method according to an embodiment of the present disclosure employs one or more of the following design features: attention; cognition; novelty; and reward. In an example embodiment, displaying the game comprises displaying both translucent and opaque moving masks to induce attention. In another example embodiment, displaying the game comprises providing a cognition inducing component selected from the group consisting of rhymes, pidgin English and homophones. In an example embodiment, displaying the game comprises interspersing repetitive language tasks with non-language mosaics. In an example embodiment, displaying the game comprises displaying motion graphics with embedded text. In an example embodiment, displaying the game comprises providing a reward comprising visual and aural feedback.

In an embodiment, the displayed game is generated in real-time based on stored game components and a stored game framework. As such, a method or system according to an embodiment of the present disclosure does not simply load a compiled game that best matches the personalized user parameters, but actually generates the game in real-time based on the stored game framework and by accessing stored game components, while modifying or completing the game components using the personalized user parameters.

In another embodiment, displaying the game further comprises modifying at least one aspect of the displayed game based on a modification in one or more of the personalized user parameters. The modification can take place before, after or during the displayed game. In a further embodiment, displaying the game further comprises modifying at least one aspect of the displayed game based on a measured result of the user in an evaluated portion of the game. In such an embodiment, modifying the at least one aspect of the displayed game is performed based on a relationship of the measured result to either: an expected result for the user based on one or more of the personalized user parameters; or a game modification threshold.

In an example embodiment, displaying the game comprises displaying fast motion graphics in addition to the auditory game objects and the visual game objects. In an example embodiment, the fast motion graphics comprise movement of about 700°/s or higher, or between about 700°/s and about 900°/s, considering the peak angular speed of the eye during a saccade reaches up to 900°/s.

In another example embodiment, the method further comprises: creating the user condition database based on providing a pre-assessment exercise to the user and generating user condition data based on the user's results of performing the pre-assessment exercise. For example, the pre-assessment exercise can be a standardized test that is administered before providing the therapeutic game in order to establish a baseline in the context of the system and method according to an embodiment of the present disclosure.

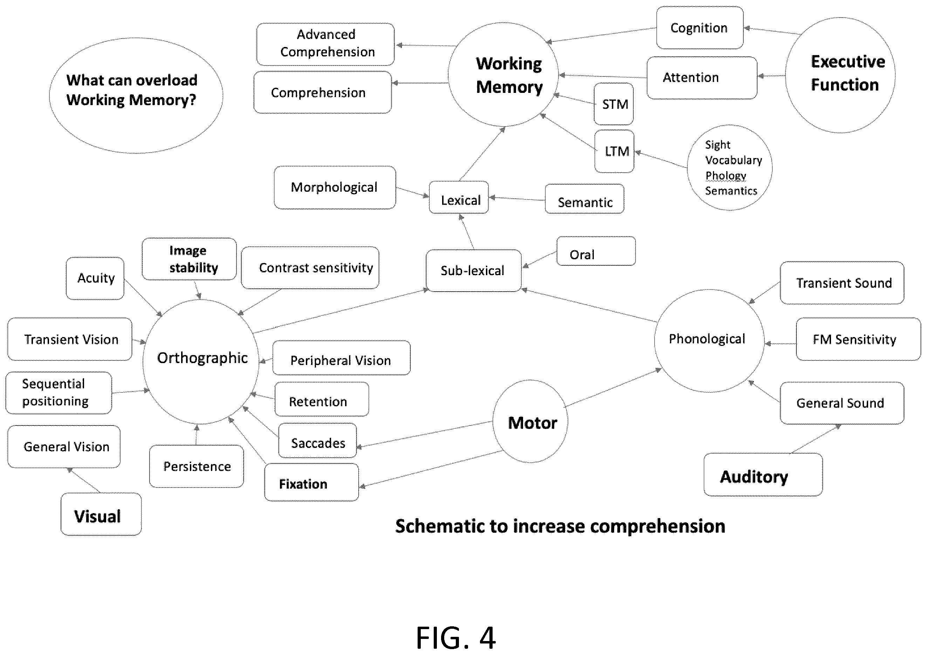

Figure 4 is a block diagram illustrating orthographic and phonological components that are targeted to improve working memory and/or executive function according to an example embodiment of the present disclosure. As outlined above, embodiments of the present disclosure display, in real-time to the user, a game including game components that concurrently provide a visual pathway challenge, an auditory pathway challenge and a cognitive pathway challenge to encourage neuroplasticity. The components illustrated in Figure 3 are examples of parameters, abilities, topics and characteristics that can be targeted by the visual pathway challenge, the auditory pathway challenge and the cognitive pathway challenge. In an example implementation: the auditory pathway challenge addresses one or more of the illustrated phonological aspects; the visual pathway challenge addresses one or more of the illustrated orthographic aspects; and the cognitive pathway challenge addresses one or more of the illustrated motor or working memory aspects.

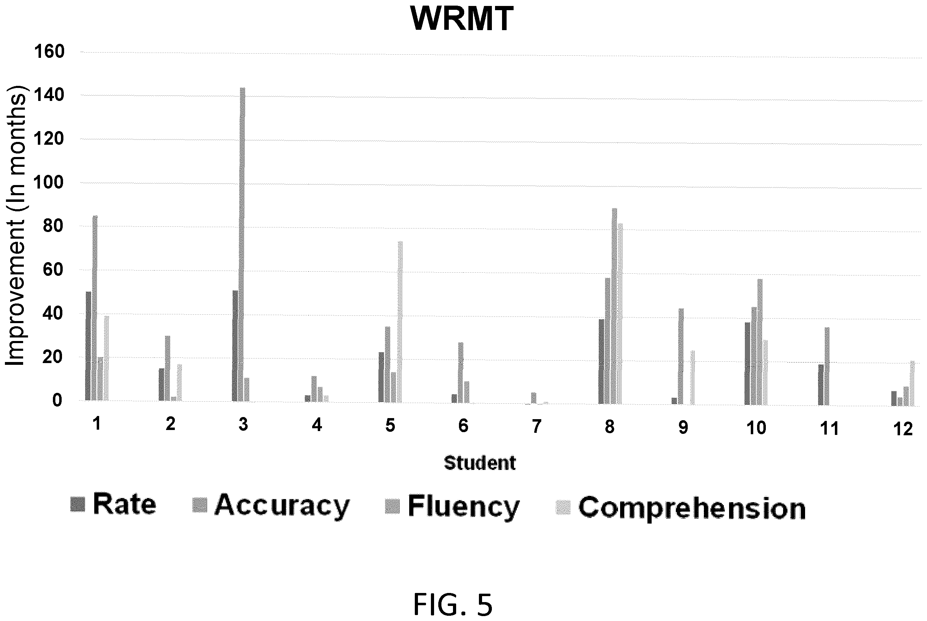

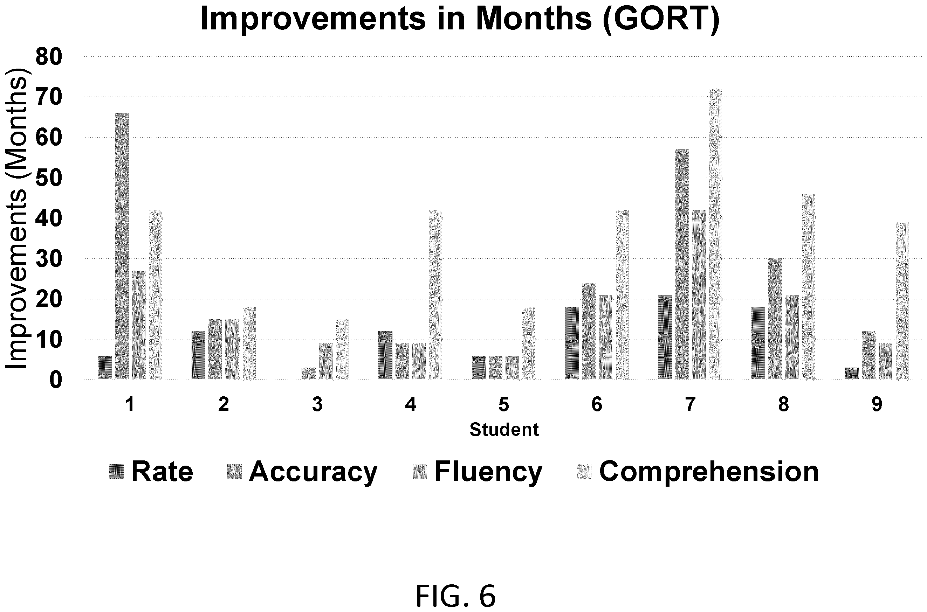

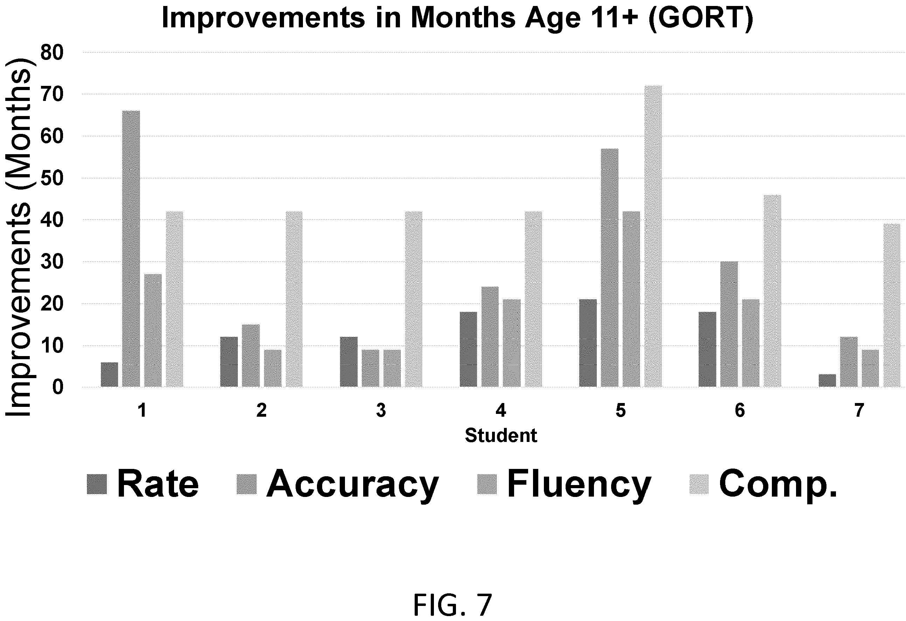

Figures 5, 6 and 7 are graphs illustrating testing improvement results from users employing a system or method according to an example embodiment of the present disclosure. For example, Figure 5 illustrates testing improvement results using the Woodcock Reading Mastery Test (WRMT). Figure 6 illustrates testing improvement results using the Gray Oral Reading Test (GORT), with Figure 7 illustrating similar results for users aged 11 and older.

Figures 5, 6 and 7 illustrate a specific example with 9 students, showing improvements for all 9 students in each of the areas of rate, accuracy, fluency and comprehension. In a first set of data (N=4), a device-based cognitive therapy program according to an embodiment of the present disclosure, which was run for 15 sessions, improved visual processing deficit (N=4). In a second set of data (N=10), a device-based cognitive therapy program according to an embodiment of the present disclosure, which was run for 15 sessions of cognitive therapy over 3-5 weeks, followed by 10 weeks of reading therapy, improved reading efficiency including word attack, word identification, reading comprehension and passage comprehension.

Examples of Games: Brief descriptions of example games according to embodiments of the present disclosure are now provided, some of which have associated figures to assist in understanding. These are non-limiting examples intended to illustrate sample implementations of different combinations of aspects of embodiments of the present disclosure.

**Bees:** Game targets eye tracking and oculomotor muscles - Right to left/ left to right targets. Targets saccadic eye movement, and tracking (best if head is kept still and just eyes are used). Conversion- because the eyes are working (looking) on the same thing. In an example implementation, front colored octagons are moving and work on (e.g. overlap/interfere with) a vestibular component that also interacts with a user's ocular eye movement. The combination of the visual components work together to help the central vision and peripheral vision together. Because the octagons are working in front of the bees, the game targets visual perception composure, which is called visual closure, which a person needs for speed reading. The brain is closing from memory and we are also working on figure ground.

**Pinball** - Auditory processing speed. Working on response speed. Auditory processing speed works out how the brain registers the information and sends the information to the cortex. The response speed is how a person responds to the message that reached the cortex. The game also has/requires visual and auditory attention because the user has to see the symbol in order to decode the symbol with phonics. The user is asked to match sound with visual. Visual closure and the movement works on the ocular vestibular interaction.

**Egypt** - Offers all the visual components but also offers a blended contrast which means the user has to be much more distinctive as to the boundaries of the letters they are seeking for. Because the text is moving, the game works on the vestibular ocular interaction with timing. Because the user is searching with their, eyes they are working on tracking. The busier the background becomes, the more the user is working on foreground and background with the same element on visual closure.

**Mosaics** - saccadic eye movement, eye tracking, visual closure, visual discrimination because of the different colors, visual placement.

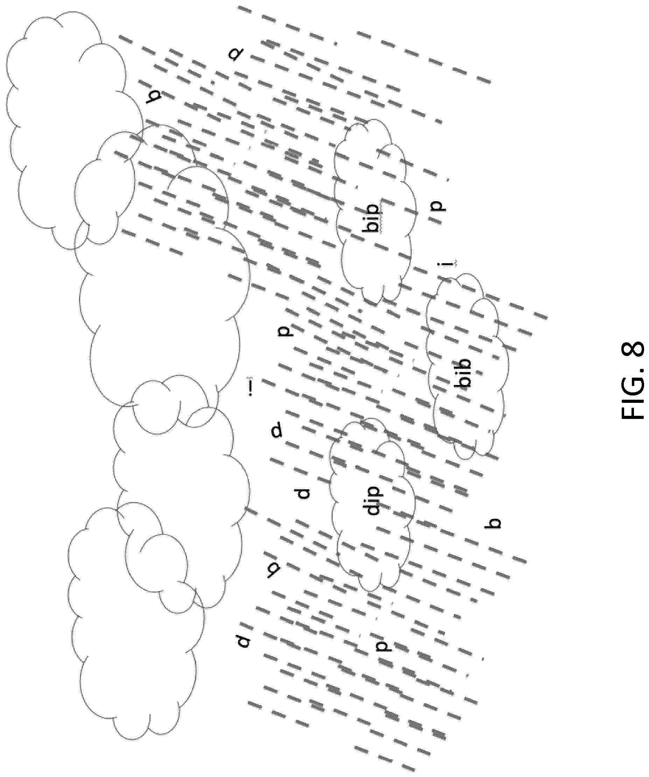

**Scotland** - decoding piece of the auditory and visual. Figures 7 and 8 illustrate example representations of an implementation of the Scotland game according to an embodiment of the present disclosure. A line drawing in Figure 7 and a screenshot in Figure 8 illustrate different game elements. The user sees something and sounds it out. Also, there is a delayed timing component which is giving the brain time to catch up with what actually has to be decoded, time to put it together. The game also includes visual foreground, visual background, and some visual memory.

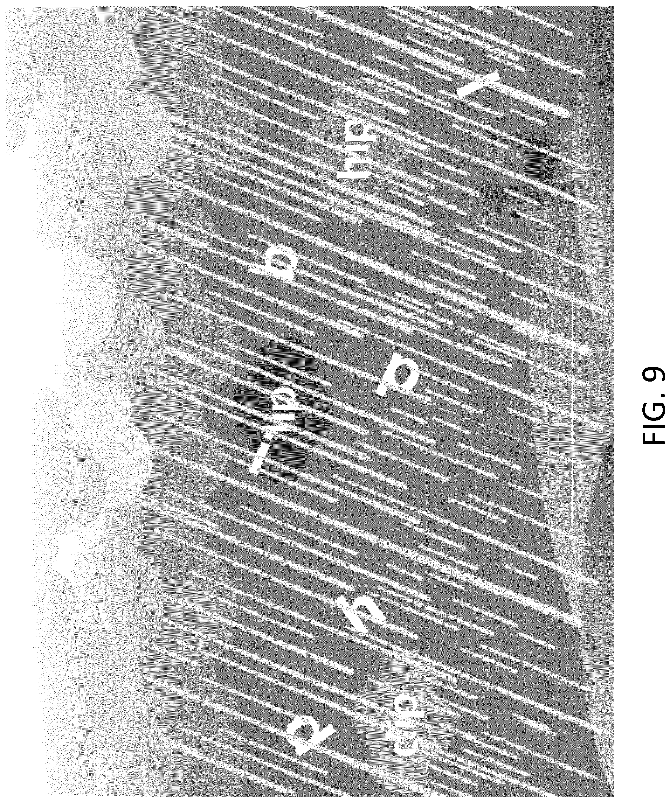

In the example as shown in Figure 8, moving clouds are provided, with text to be found and placed in the top cloud. Tumbling characters are provided and visually mingled with falling rain drops with differentially recorded sound for auditory stimulation. The screenshot of Figure 9 also shows three blanks in which the letters of the word "lip" shown in the top cloud are to be placed by the user; in the screenshot of Figure 9, the "I" can be seen near the bottom left of the screen, the "i" can be seen overlapping with the top cloud in which the "target" word is provided, and the "p" can be seen above the cloud that includes the word "dip". In an example implementation, the visual background is the sky and the clouds, the visual foreground is the falling rain, with the letters being provided in a way that appears to be between the visual foreground and the visual background. The falling raindrops comprise a moving mask, which can be translucent and/or opaque, to induce attention. The moving clouds, tumbling characters and falling raindrops can all be considered to provide motion graphics, with embedded text provided in the displayed scene. The example game addresses orthographic aspects (e.g. letters and spelling) and comprises a visual pathway challenge, as well as a cognitive pathway challenge by having the elements displayed for a brief period of time.

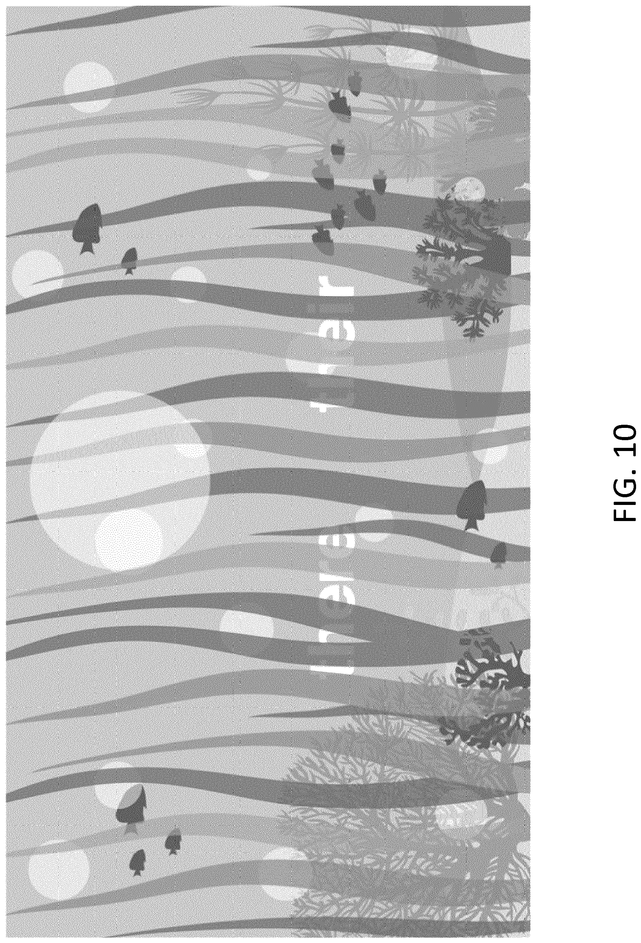

**Australia** - homophones work very much on the critical skills of active working memory, which will give a user a longer working memory span. When a user is manipulating something in their mind while contemplating something different, it is also very important for the overlay into cognitive flexibility. The user is able to focus on something and shift their mind to something else and then come back to it. The game then also works on the automaticity of the decoding skill which works visually and auditory. In the example embodiment shown in Figure 10, an underwater scene is provided, in which the homophones "there" and "their" are displayed in a manner that appears to be "behind" the seaweed and other sea vegetation, and "in front of" the water background. In an example embodiment, the seaweed has visual movement simulating motion, which causes the user's mind to focus on the movement while trying to focus on and decode the displayed homophones. In another example embodiment, one or more of the seaweed, the fish, and the bubbles have visual movement simulating motion, with the game being adapted to add more movement of an increasing number of elements for an increased skill/assessment level. The seaweed, fish and bubbles can comprise a moving mask, which can be translucent and/or opaque, to induce attention. The example game addresses orthographic aspects (e.g. letters and spelling) and comprises a visual pathway challenge, as well as a cognitive pathway challenge by having the elements displayed for a brief period of time.

For younger users, text can be replaced by pictures, which creates an overlay from the right brain to the left brain. This gives younger users an easier transition to lexicon which creates more meaning in the user's brain and also makes it easier to bridge from picture thinking to lexicon thinking.

**China** - Auditory processing gives the ear a chance to hear what they need to hear by long gating the word and then closing the sounds to correct words.

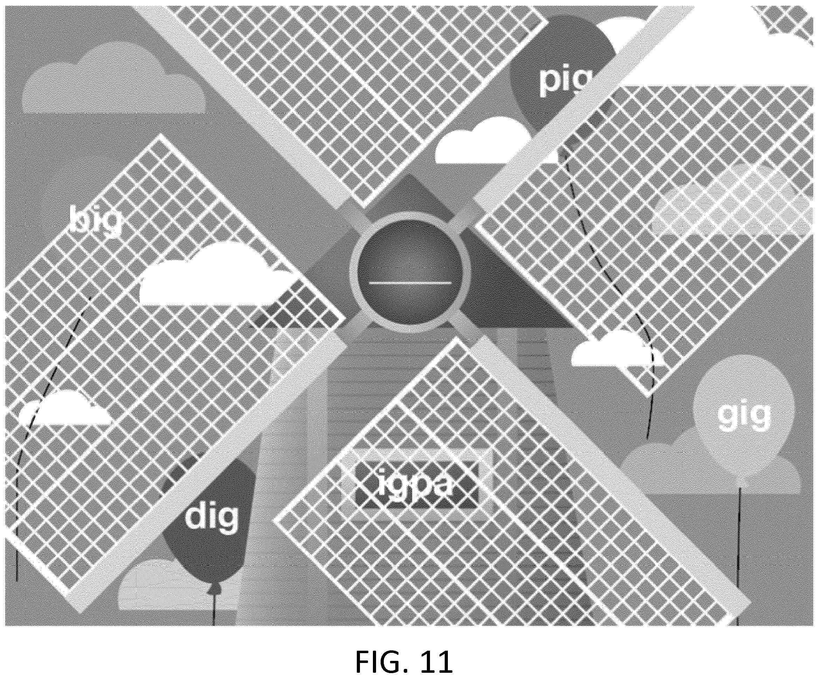

**Holland** - Visual closure, pig Latin working memory, word attack, word recognition, decoding and comprehension. As shown in the example embodiment of Figure 11, this game provides different visual elements including a visual background of the sky and clouds, and a visual foreground of the windmill blades/vanes. A plurality of balloons are provided, each with letters forming words, in this case either in English or pig Latin. A variety of implementations can employ this design. In operation of the game, the blades/vanes of the windmill appear to move, causing the user to focus on the words/letters in the balloons and in other areas of the display to decode them despite the visual "interference" of the other game elements. In another example embodiment, one or more of the balloons and windmill blades have visual movement simulating motion, with the game being adapted to add more movement of an increasing number of elements for an increased skill/assessment level. The balloons and windmill blades can comprise a moving mask, which can be translucent and/or opaque, to induce attention. The example game addresses orthographic aspects (e.g. letters and spelling) and comprises a visual pathway challenge, as well as a cognitive pathway challenge by having the elements displayed for a brief period of time.

Examples were undertaken for young children up to 8 years old with 15 one-hour sessions to complete within 5 weeks. In this example, every correct answer gets the same 2 or 3 points regardless how many attempts they take to get the correct answer. A user or client gets bonus points if the correct answer is completed within a time factor starting at 5 seconds and working up to 8 seconds. Bonus points started at 1 point and worked up to 6 points.

For younger users, the following modifications were implemented:
Bees - honeycombs are easier (2 ½ minutes to 4 minutes allotted length of time)
Pinball - speed of the balls are slower (8 ½ minutes length of time)
Egypt - all the words to the sentences are in each line. (5 minutes in length of time)
Sunny Scotland - letter and sound association is different. Spelling is easier.(10 min)
Australia - pictures are present for all 15 sessions. (8 ½ minutes length of time)
China - is the same. (8 ½ minutes length of time)
Holland - words are easier. (5 minutes in length of time)

Examples were also undertaken for children 8 years old and up, who have some reading skills. Again, there were 15 one-hour sessions to complete within 5 weeks, with the same point and bonus point structure.

Compared to the younger exercises, the following differences exist:
Bees - honeycombs increase in difficulty (2 ½ minutes to 4 minutes in length of time)
Pinball - speed of the balls are faster (8 ½ minutes in length of time)

In an example implementation, 100% of students were observed to see changes in reading behaviors. For example, an average of a 2 grade level improvement was observed.

It is said that one in 10 people have some form of learning differences (LD), including dyslexia. 75% of people who do not complete school may have a LD. LD are strongly associated with stress, anxiety, depression and suicidal tendencies. 67% of people with ADHD have LD.42 % of people with learning differences are unemployed; those who are employed make 50% less than their peers. Multi-generation poverty is associated with lack of literacy.

Embodiments of the present disclosure provide one or more of the following advantages: leverage the power of engagement with exciting gameplays, points system and fun graphics; boost phonemic awareness by separating words into individual sounds and gradually introducing blends; use a targeted multisensory approach to create connections between the auditory and visual pathways required for fluent reading; process oriented gameplays specifically designed to strengthen coding and decoding skills; improve eye movement control with gameplays designed to strengthen eye tracking in all directions; and help improve executive functioning skills through multiple concurrent or simultaneous demands, performance feedback and problem solving.

Embodiments of the present disclosure can assist with providing cognitive therapy or learning development to address one or more of the following: for children, reading age appears to have reached a plateau and falls further and further behind with each passing year, or for adults struggled with reading or keeping up with peers in school; diagnosed with dyslexia or a learning disorder; have trouble picking up a skill due to ADHD or ASD; difficulty carryout out oral instructions; poor reading, spelling or writing skills; good reading skills, but can't recall what they have read; reading skills are normal, but feel discomfort or suffer from fatigue when reading; poor working memory; feel uncomfortable looking at black letters on white paper, or white letters on a blackboard; and have eye movement control problems. In an example embodiment, a system and method provide a device-based multi-sensory therapeutic game adapted to provide therapy for attention deficit hyperactivity disorder (ADHD).

In an example embodiment, personalized device-based cognitive therapy is delivered in the following format and context: pre-assessment; 15 one-hour computer-based training sessions, ideally completed in 3-5 weeks; 3 hours/week of reading for 10 weeks, which can be done at home; and a post-assessment.

Embodiments of the present disclosure provide one or more of the following advantages: improve auditory processing speed; improve visual processing speed; bond auditory and visual functions; increase working memory capacity; improve attention; improve motivation; and improve eye movement control.

Embodiments of the present disclosure provide benefits in one or more of the following environments: educational institutions, such as elementary schools, middle schools, secondary/high schools, colleges, or universities; hospital facilities dealing with conditions such as intellectual disability, attention deficit hyperactivity disorder (ADHD), stress, anxiety, depression etc.; private medical/healthcare practitioners and their clinics, such as clinical psychologists, occupational therapists (OTs), vision therapists, reading clinics; employment readiness programs, such as learning based skill development.

In the preceding description, for purposes of explanation, numerous details are set forth in order to provide a thorough understanding of the embodiments. However, it will be apparent to one skilled in the art that these specific details are not required. In other instances, well-known electrical structures and circuits are shown in block diagram form in order not to obscure the understanding. For example, specific details are not provided as to whether the embodiments described herein are implemented as a software routine, hardware circuit, firmware, or a combination thereof.

Embodiments of the disclosure can be represented as a computer program product stored in a machine-readable medium (also referred to as a computer-readable medium, a processor-readable medium, or a computer usable medium having a computer-readable program code embodied therein). The machine-readable medium can be any suitable tangible, non-transitory medium, including magnetic, optical, or electrical storage medium including a diskette, compact disk read only memory (CD-ROM), memory device (volatile or non-volatile), or similar storage mechanism. The machine-readable medium can contain various sets of instructions, code sequences, configuration information, or other data, which, when executed, cause a processor to perform steps in a method according to an embodiment of the disclosure. Those of ordinary skill in the art will appreciate that other instructions and operations necessary to implement the described implementations can also be stored on the machine-readable medium. The instructions stored on the machine-readable medium can be executed by a processor or other suitable processing device, and can interface with circuitry to perform the described tasks.

The above-described embodiments are intended to be examples only. Alterations, modifications and variations can be effected to the particular embodiments by those of skill in the art without departing from the scope, which is defined solely by the claims appended hereto.

## Claims

1. A system (130) for providing a device-based multi-sensory therapeutic game or a device-based multi-sensory learning development tool, the system (130) comprising:
a component provision module (140), in communication with a database (120) which stores personalized user parameters associated with a condition of a user, configured to collect in real-time personalized user parameters to be stored in the database (120), predict impairments or improvements of the user by applying artificial intelligence to both the collected and stored personalized user parameters, and provide, based on the artificial intelligence predictions, game components and a game generation framework stored in the database (120);
a generator module (150), in communication with the component provision module (140), configured to receive all the personalized user parameters from the component provision module (140) and generate, in real-time and using the game generation framework in conjunction with the database (120), scenes and assets including auditory game objects and visual game objects based on the received personalized user parameters; and
a display module (160) configured to display, in real-time to the user (110), a game that concurrently provides a visual pathway challenge, an auditory pathway challenge and a cognitive pathway challenge to encourage neuroplasticity, the game including the game components, the auditory game objects and the visual game objects, and further configured to dynamically modify at least one aspect of the displayed game based on a modification in one or more of the personalized user parameters stored in the database (120) before displaying the game and/or the personalized user parameters collected by the component provision module (140) in real-time while displaying the game.

2. The system of claim 1 wherein the game components displayed by the display module comprise generic game components that are presented in a similar manner to any user, and the visual game objects and the auditory game objects comprise components that are based on personalized user parameters, and are presented in a different manner to different users depending on the associated personalized user parameters.

3. The system of claim 1 wherein the component provision module, the generator module and the display module cooperate to provide the device-based multi-sensory therapeutic game adapted to provide therapy for attention deficit hyperactivity disorder (ADHD).

4. The system of claim 1 wherein the scenes and assets including the auditory game objects and the visual game objects are generated independent of a language of game delivery.

5. The system of claim 1 wherein the game components, the auditory game objects and the visual game objects are provided in a first language to concurrently challenge the user's visual pathway, auditory pathway and cognitive pathway in the first language.

6. The system of claim 1 wherein the game components, the auditory game objects and the visual game objects are provided in a first language to concurrently challenge the user's visual pathway, auditory pathway and cognitive pathway in a second language, the second language being different from the first language.

7. A processor-implemented method for providing a device-based multi-sensory therapeutic game or a device-based multi-sensory learning development tool, the method comprising:
obtaining, from a database (120), personalized user parameters associated with a condition of the user for which the multi-sensory therapeutic game is designed to provide therapy;
collecting in real-time personalized user parameters to be stored in the database (120);
predicting impairments or improvements of the user by applying artificial intelligence to both the collected and stored personalized user parameters;
providing, based on the artificial intelligence predictions, game components and a game generation framework stored in the database (120);
generating, in real-time and using the game generation framework in conjunction with the database (120), scenes and assets including auditory game objects and visual game objects based on both the collected and stored personalized user parameters; and
displaying, in real-time to the user, a game that concurrently provides a visual pathway challenge, an auditory pathway challenge and a cognitive pathway challenge to encourage neuroplasticity, the game including the auditory game objects and the visual game objects; and dynamically modifying at least one aspect of the displayed game based on a modification in one or more of the personalized user parameters stored in the database (120) before displaying the game and/or the personalized user parameters collected in real-time while displaying the game.

8. The method of claim 7 wherein the displayed game components comprise generic game components that are presented in a similar manner to any user, and the displayed visual game objects and the displayed auditory game objects comprise components that are based on personalized user parameters, and are presented in a different manner to different users depending on the associated personalized user parameters.

9. The method of claim 7 wherein displaying the game comprises one of the following:
simultaneously providing the visual pathway challenge, the auditory pathway challenge, and the cognitive pathway challenge;
displaying both translucent and opaque moving masks to induce attention;
providing a cognition inducing component selected from the group consisting of rhymes, pidgin English and homophones;
interspersing repetitive language tasks with non-language mosaics;
displaying motion graphics with embedded text;
providing a reward comprising visual and aural feedback;
modifying at least one aspect of the displayed game based on a measured result of the user in an evaluated portion of the game;
modifying at least one aspect of the displayed game based on a relationship of a measured result of the user in an evaluated portion of the game to either: an expected result for the user based on one or more of the personalized user parameters, or a game modification threshold; displaying fast motion graphics in addition to the auditory game objects and the visual game objects.

10. The method of claim 9 wherein simultaneously providing the visual pathway challenge, the auditory pathway challenge, and the cognitive pathway challenge comprises using complete words and their corresponding sounds.

11. The method of claim 7 wherein the game components, the auditory game objects and the visual game objects are displayed in a first language to concurrently challenge the user's visual pathway, auditory pathway and cognitive pathway in a second language, the second language being different from the first language.

## Patentansprüche

1. System (130) zum Bereitstellen eines vorrichtungsbasierten multisensorischen therapeutischen Spiels oder eines vorrichtungsbasierten multisensorischen Tools zur Lernentwicklung, wobei das System (130) Folgendes umfasst:
ein Komponentenbereitstellungsmodul (140), das mit einer Datenbank (120) in Verbindung steht, die personalisierte Benutzerparameter speichert, die mit einem Zustand eines Benutzers verknüpft sind, dazu konfiguriert, in Echtzeit personalisierte Benutzerparameter zu sammeln, die in der Datenbank (120) zu speichern sind, Beeinträchtigungen oder Verbesserungen des Benutzers durch Anwendung künstlicher Intelligenz sowohl auf die gesammelten als auch auf die gespeicherten personalisierten Benutzerparameter vorherzusagen und basierend auf den Vorhersagen der künstlichen Intelligenz Spielkomponenten und ein Framework zur Spielgenerierung bereitzustellen, die in der Datenbank (120) gespeichert sind;
ein Generatormodul (150), das mit dem Komponentenbereitstellungsmodul (140) in Verbindung steht und dazu konfiguriert ist, alle personalisierten Benutzerparameter vom Komponentenbereitstellungsmodul (140) zu empfangen und in Echtzeit und unter Verwendung des Frameworks zur Spielgenerierung zusammen mit der Datenbank (120) Szenen und Assets zu generieren, einschließlich auditiver Spielobjekte und visueller Spielobjekte, basierend auf den empfangenen personalisierten Benutzerparametern; und
ein Display-Modul (160), das dazu konfiguriert ist, dem Benutzer (110) in Echtzeit ein Spiel anzuzeigen, das gleichzeitig eine visuelle Pfadherausforderung, eine auditive Pfadherausforderung und eine kognitive Pfadherausforderung bereitstellt, um die Neuroplastizität zu fördern, wobei das Spiel die Spielkomponenten, die auditiven Spielobjekte und die visuellen Spielobjekte einschließt, und ferner dazu konfiguriert ist, mindestens einen Aspekt des angezeigten Spiels basierend auf einer Modifizierung in einem oder mehreren der in der Datenbank (120) gespeicherten personalisierten Benutzerparameter vor der Anzeige des Spiels und/oder der vom Komponentenbereitstellungsmodul (140) gesammelten personalisierten Benutzerparameter in Echtzeit während der Anzeige des Spiels dynamisch zu modifizieren.

2. System nach Anspruch 1, wobei die Spielkomponenten, die durch das Display-Modul angezeigt werden, generische Spielkomponenten umfassen, die jedem Benutzer auf ähnliche Weise präsentiert werden, und die visuellen Spielobjekte und die auditiven Spielobjekte Komponenten umfassen, die auf personalisierten Benutzerparametern basieren und unterschiedlichen Benutzern auf unterschiedliche Weise präsentiert werden, je nach den verknüpften personalisierten Benutzerparametern.

3. System nach Anspruch 1, wobei das Komponentenbereitstellungsmodul, das Generatormodul und das Anzeigemodul zusammenwirken, um das vorrichtungsbasierte multisensorische therapeutische Spiel bereitzustellen, das zur Bereitstellung einer Therapie für die Aufmerksamkeitsdefizit-/Hyperaktivitätsstörung (ADHS) angepasst ist.

4. System nach Anspruch 1, wobei die Szenen und Assets, einschließlich der auditiven Spielobjekte und der visuellen Spielobjekte, unabhängig von einer Sprache der Spieldarbietung generiert werden.

5. System nach Anspruch 1, wobei die Spielkomponenten, die auditiven Spielobjekte und die visuellen Spielobjekte in einer ersten Sprache bereitgestellt werden, um gleichzeitig den visuellen Pfad, den auditiven Pfad und den kognitiven Pfad des Benutzers in der ersten Sprache herauszufordern.

6. System nach Anspruch 1, wobei die Spielkomponenten, die auditiven Spielobjekte und die visuellen Spielobjekte in einer ersten Sprache bereitgestellt werden, um gleichzeitig den visuellen Pfad, den auditiven Pfad und den kognitiven Pfad des Benutzers in einer zweiten Sprache herauszufordern, wobei sich die zweite Sprache von der ersten Sprache unterscheidet.

7. Prozessorimplementiertes Verfahren zum Bereitstellen eines vorrichtungsbasierten multisensorischen therapeutischen Spiels oder eines vorrichtungsbasierten multisensorischen Tools zur Lernentwicklung, wobei das Verfahren Folgendes umfasst:
Erhalten von personalisierten Benutzerparametern aus einer Datenbank (120), die mit einem Zustand des Benutzers verknüpft sind, für den das multisensorische therapeutische Spiel zur Bereitstellung einer Therapie ausgelegt ist;
Sammeln von personalisierten Benutzerparametern in Echtzeit, die in der Datenbank (120) zu speichern sind; Vorhersagen von Beeinträchtigungen oder Verbesserungen des Benutzers durch Anwenden künstlicher Intelligenz sowohl auf die gesammelten als auch auf die gespeicherten personalisierten Benutzerparameter;
Bereitstellen von Spielkomponenten und eines in der Datenbank (120) gespeicherten Frameworks zur Spielgenerierung basierend auf den Vorhersagen der künstlichen Intelligenz;
Generieren von Szenen und Assets, einschließlich auditiver Spielobjekte und visueller Spielobjekte, in Echtzeit und unter Verwendung des Frameworks zur Spielgenerierung zusammen mit der Datenbank (120), basierend sowohl auf den gesammelten als auch auf den gespeicherten personalisierten Benutzerparametern; und
Anzeigen eines Spiels in Echtzeit für den Benutzer, das gleichzeitig eine visuelle Pfadherausforderung, eine auditive Pfadherausforderung und eine kognitive Pfadherausforderung bereitstellt, um die Neuroplastizität zu fördern, wobei das Spiel die auditiven Spielobjekte und die visuellen Spielobjekte einschließt; und dynamisches Modifizieren mindestens eines Aspekts des angezeigten Spiels basierend auf einer Modifizierung in einem oder mehreren der personalisierten Benutzerparameter, die in der Datenbank (120) gespeichert sind, bevor das Spiel angezeigt wird, und/oder der personalisierten Benutzerparameter, die in Echtzeit gesammelt werden, während das Spiel angezeigt wird.

8. Verfahren nach Anspruch 7, wobei die angezeigten Spielkomponenten generische Spielkomponenten umfassen, die jedem Benutzer auf ähnliche Weise präsentiert werden, und die angezeigten visuellen Spielobjekte und die angezeigten auditiven Spielobjekte Komponenten umfassen, die auf personalisierten Benutzerparametern basieren und unterschiedlichen Benutzern auf unterschiedliche Weise präsentiert werden, je nach den verknüpften personalisierten Benutzerparametern.

9. Verfahren nach Anspruch 7, wobei das Anzeigen des Spiels eines von Folgendem umfasst: gleichzeitiges Bereitstellen der visuellen Pfadherausforderung, der auditiven Pfadherausforderung und der kognitiven Pfadherausforderung;
Anzeigen von sowohl durchscheinenden als auch lichtundurchlässigen beweglichen Masken, um Aufmerksamkeit zu induzieren;
Bereitstellen einer kognitionsinduzierenden Komponente, die aus der Gruppe ausgewählt wird, die aus Reimen, Pidgin-Englisch und Homophonen besteht;
Einstreuen sich wiederholender Sprachaufgaben mit nichtsprachlichen Mosaiken;
Anzeigen von Bewegungsgrafiken mit eingebettetem Text;
Bereitstellen einer Belohnung, umfassend visuelles und akustisches Feedback;
Modifizieren mindestens eines Aspekts des angezeigten Spiels basierend auf einem gemessenen Ergebnis des Benutzers in einem bewerteten Teil des Spiels;
Modifizieren mindestens eines Aspekts des angezeigten Spiels basierend auf einer Beziehung eines gemessenen Ergebnisses des Benutzers in einem bewerteten Teil des Spiels zu entweder: einem erwarteten Ergebnis für den Benutzer basierend auf einem oder mehreren der personalisierten Benutzerparameter oder einem Schwellenwert für die Spielmodifizierung;
Anzeigen von Schnellbewegungsgrafiken zusätzlich zu den akustischen Spielobjekten und den visuellen Spielobjekten.

10. Verfahren nach Anspruch 9, wobei das gleichzeitige Bereitstellen der visuellen Pfadherausforderung, der auditiven Pfadherausforderung und der kognitiven Pfadherausforderung das Verwenden vollständiger Wörter und ihrer entsprechenden Laute umfasst.

11. Verfahren nach Anspruch 7, wobei die Spielkomponenten, die auditiven Spielobjekte und die visuellen Spielobjekte in einer ersten Sprache angezeigt werden, um gleichzeitig den visuellen Pfad, den auditiven Pfad und den kognitiven Pfad des Benutzers in einer zweiten Sprache herauszufordern, wobei sich die zweite Sprache von der ersten Sprache unterscheidet.

## Revendications

1. Système (130) permettant de fournir un jeu thérapeutique multisensoriel basé sur un dispositif ou un outil de développement d'apprentissage multisensoriel basé sur un dispositif, le système (130) comprenant :
un module de fourniture de composants (140), en communication avec une base de données (120) qui stocke des paramètres utilisateur personnalisés associés à une condition d'un utilisateur, conçu pour collecter en temps réel des paramètres utilisateur personnalisés à stocker dans la base de données (120), prédire les déficiences ou les améliorations de l'utilisateur en appliquant l'intelligence artificielle aux paramètres utilisateur personnalisés à la fois collectés et stockés, et fournir, en fonction des prédictions de l'intelligence artificielle, des composants de jeu et un cadre de génération de jeu stockés dans la base de données (120) ;
un module générateur (150), en communication avec le module de fourniture de composants (140), conçu pour recevoir tous les paramètres utilisateur personnalisés du module de fourniture de composants (140) et générer, en temps réel et à l'aide du cadre de génération de jeu en conjonction avec la base de données (120), des scènes et des ressources comportant des objets de jeu auditifs et des objets de jeu visuels en fonction des paramètres utilisateur personnalisés reçus ; et
un module d'affichage (160) conçu pour afficher, en temps réel à l'utilisateur (110), un jeu qui offre simultanément un défi de parcours visuel, un défi de parcours auditif et un défi de parcours cognitif pour encourager la neuroplasticité, le jeu comportant les composants de jeu, les objets de jeu auditifs et les objets de jeu visuels, et en outre conçu pour modifier dynamiquement au moins un aspect du jeu affiché en fonction d'une modification d'un ou plusieurs des paramètres utilisateur personnalisés stockés dans la base de données (120) avant d'afficher le jeu et/ou des paramètres utilisateur personnalisés collectés par le module de fourniture de composants (140) en temps réel lors de l'affichage du jeu.

2. Système selon la revendication 1, dans lequel les composants de jeu affichés par le module d'affichage comprennent des composants de jeu génériques qui sont présentés de manière similaire à une quelconque utilisateur, et les objets de jeu visuels et les objets de jeu auditifs comprennent des composants qui sont basés sur des paramètres utilisateur personnalisés, et sont présentés de manière différente à différents utilisateurs en fonction des paramètres utilisateur personnalisés associés.

3. Système selon la revendication 1, dans lequel le module de fourniture de composants, le module générateur et le module d'affichage coopèrent pour fournir le jeu thérapeutique multisensoriel basé sur un dispositif adapté pour fournir une thérapie pour le trouble déficitaire de l'attention avec hyperactivité (TDAH).

4. Système selon la revendication 1, dans lequel les scènes et les ressources comportant les objets de jeu auditifs et les objets de jeu visuels sont générés indépendamment d'une langue de diffusion du jeu.

5. Système selon la revendication 1, dans lequel les composants de jeu, les objets de jeu auditifs et les objets de jeu visuels sont fournis dans une première langue pour défier simultanément le parcours visuel, le parcours auditif et le parcours cognitif de l'utilisateur dans la première langue.

6. Système selon la revendication 1, dans lequel les composants de jeu, les objets de jeu auditifs et les objets de jeu visuels sont fournis dans une première langue pour défier simultanément le parcours visuel, le parcours auditif et le parcours cognitif de l'utilisateur dans une seconde langue, la seconde langue étant différente de la première langue.

7. Procédé mis en œuvre par un processeur pour fournir un jeu thérapeutique multisensoriel basé sur un dispositif ou un outil de développement d'apprentissage multisensoriel basé sur un dispositif, le procédé comprenant :
l'obtention, à partir d'une base de données (120), de paramètres utilisateur personnalisés associés à une condition de l'utilisateur pour laquelle le jeu thérapeutique multisensoriel est conçu pour fournir une thérapie ;
la collecte en temps réel de paramètres utilisateur personnalisés à stocker dans la base de données (120) ; la prédiction des déficiences ou des améliorations de l'utilisateur en appliquant l'intelligence artificielle aux paramètres utilisateur personnalisés à la fois collectés et stockés ;
la fourniture, en fonction des prédictions de l'intelligence artificielle, de composants de jeu et d'un cadre de génération de jeu stockés dans la base de données (120) ;
la génération, en temps réel et à l'aide du cadre de génération de jeu en conjonction avec la base de données (120), de scènes et de ressources comportant des objets de jeu auditifs et des objets de jeu visuels en fonction des paramètres utilisateur personnalisés à la fois collectés et stockés ; et
l'affichage, en temps réel à l'utilisateur, d'un jeu qui offre simultanément un défi de parcours visuel, un défi de parcours auditif et un défi de parcours cognitif pour encourager la neuroplasticité, le jeu comportant les objets de jeu auditifs et les objets de jeu visuels ; et la modification dynamique d'au moins un aspect du jeu affiché en fonction d'une modification d'un ou de plusieurs des paramètres utilisateur personnalisés stockés dans la base de données (120) avant l'affichage du jeu et/ou des paramètres utilisateur personnalisés collectés en temps réel lors de l'affichage du jeu.

8. Procédé selon la revendication 7, dans lequel les composants de jeu affichés comprennent des composants de jeu génériques qui sont présentés d'une manière similaire à un quelconque utilisateur, et les objets de jeu visuels affichés et les objets de jeu auditifs affichés comprennent des composants qui sont basés sur des paramètres utilisateur personnalisés, et sont présentés d'une manière différente à différents utilisateurs en fonction des paramètres utilisateur personnalisés associés.

9. Procédé selon la revendication 7, dans lequel l'affichage du jeu comprend l'une des opérations suivantes : fourniture simultanée du défi de parcours visuel, du défi de parcours auditif et du défi de parcours cognitif ;
l'affichage de masques mobiles translucides et opaques pour attirer l'attention ;
la fourniture d'un composant induisant la cognition sélectionné dans le groupe constitué de rimes, anglais pidgin et homophones ;
l'intercalation de tâches langagières répétitives avec des mosaïques non linguistiques ;
l'affichage de graphiques en mouvement avec du texte intégré ;
la fourniture d'une récompense comprenant un retour visuel et auditif ;
la modification d'au moins un aspect du jeu affiché en fonction d'un résultat mesuré de l'utilisateur dans une partie évaluée du jeu ;
la modification d'au moins un aspect du jeu affiché en fonction d'une relation entre un résultat mesuré de l'utilisateur dans une partie évaluée du jeu et soit : un résultat attendu pour l'utilisateur en fonction d'un ou plusieurs des paramètres utilisateur personnalisés, soit un seuil de modification du jeu ;
l'affichage des graphiques en mouvement rapide en plus des objets de jeu auditifs et des objets de jeu visuels.

10. Procédé selon la revendication 9, dans lequel la fourniture simultanée du défi de parcours visuel, du défi de parcours auditif et du défi de parcours cognitif comprennent l'utilisation de mots complets et de leurs sons correspondants.

11. Procédé selon la revendication 7, dans lequel les composants de jeu, les objets de jeu auditifs et les objets de jeu visuels sont affichés dans une première langue pour défier simultanément le parcours visuel, le parcours auditif et le parcours cognitif de l'utilisateur dans une seconde langue, la seconde langue étant différente de la première langue.
